# EUROPEAN PATENT SPECIFICATION

(11) **EP 2 004 554 B1**
(45) Date of publication and mention of the grant of the patent: **23.09.2015**
(21) Application number: 07732256.8
(22) Date of filing: 02.04.2007
(51) Int. Cl.: C02F 1/32

(54) **FLUID TREATMENT APPARATUS COMPRISING ULTRAVIOLET LIGHT EMITTING DIODE**
FLUIDBEHANDLUNGSVORRICHTUNG MIT UV-LICHT EMITTIERENDER DIODE
APPAREIL DE TRAITEMENT DE FLUIDE COMPRENANT DES DIODES ÉLECTROLUMINESCENTES À RAYONNEMENT ULTRAVIOLET

(30) Priority: 01.04.2006 GB 0606604
(43) Date of publication of application: 24.12.2008
(73) Proprietor: P.W. Circuits Limited, South Wigston, Leicester LE18 4PN (GB)
(72) Inventor: MASON, Colin, Leicester LE18 4PN (GB); O'CONNOR, Cecil, Leicester LE18 4PN (GB)
(74) Representative: Oxley, Robin John George
(86) International application number: PCT/GB2007/001205
(87) International publication number: WO 2007/113537

(56) References cited:
- WO-A-02/12127
- WO-A-02/083570
- WO-A-03/035145
- WO-A-03/096387
- US-A- 5 891 329
- US-A1- 2003 170 151
- US-B1- 6 787 782
- Regina Sommer ET AL: "UV Drinking Water Disinfection - Requirements, Testing and Surveillance: Exemplified by the austrian National Standards M 5873-3 and M 5873-2", , 1 December 2004 (2004-12-01), pages 27-35, XP55034740, Retrieved from the Internet: URL:http://iuva.org/sites/default/files/me mber/news/IUVA_news/Vol06/Issue4/060404Som meretalArticle.pdf [retrieved on 2012-08-06]

## Description

The present invention relates to treatment apparatus and more particularly treatment apparatus for at least partially disinfecting a fluid such as air or water. More specifically the present invention relates to treatment apparatus for disinfecting water in aquatic environments such as aquariums, fish ponds or the like.

The use of UV light in the sterilisation of water is a well known. UV light disinfects water by permanently deactivating organisms such as bacteria, spores, moulds, viruses or the like. Light having wavelengths between 200nm and 300nm, also known as UVC, is known to be responsible for this effect. Typically, the most effective wavelength is of the order 265nm, although other wavelengths are known to be more effective against particular organisms. Unlike chemical disinfectants, organisms are unable to develop immune mechanisms against UV.

This application of UV sterilisation in aquatic environments such as ponds or aquariums is also known. Typically, a submersible tube is provided for immersion in a pond or aquarium. Such tubes generally employ a low-pressure mercury vapour discharge lamp surrounded by a water jacket whose inner wall is made from a UVC-transparent quartz material. The lamp generally produces a UV radiation of wavelength in the region 254nm, and visible light.

However, UV lamps and tubes are relatively high power consumption, requiring a mains power supply. Hence, for electrical safety reasons, submersible UV lamps have to be highly water resistant to an ingress protection rating of IP68 or higher, thus making them expensive to manufacture.

UV lamps and tubes also degrade over time and eventually become ineffective for water treatment making replacement necessary. This adds significantly to the costs of UV water treatment, both because of the relatively high cost of the new tubes, and because of the frequency of replacement. Furthermore, UV degradation is not immediately obvious to an observer. Hence, treatment lamps and tubes are often used for a long time after they become ineffective. To mitigate this problem some treatment apparatus is provided with a clock for recording the cumulative length of time the lamp is used for, thereby providing an indication of when the lamp or tube should be replaced. However, such clocks are based on an average degradation time for the tubes, rather than any direct indication of tube performance.

UV lamps and tubes are also relatively large, and therefore, take up significant proportion of the available space in small fish tanks ponds or the like. They also rely on pump induced circulation of the water around the tank or pond to ensure effective treatment. In small tanks with good circulation this is not a significant problem, but in larger aquatic environments, such as larger aquariums, ponds or the like, a distributed arrangement of a plurality of treatment tubes can become necessary.

Examples of known UV fluid treatment systems are disclosed in published US patent application US2003/0170151 which discloses a UV system for treating fluid in an air conditioning system using flashing UV LED's and in published international patent application WO2/12127 which uses control circuitry to control the power of the LED in a water treatment system, the control of power may include pulse width modulation of the control signal.

The object of the present invention is to provide treatment apparatus, which mitigates at least one of the above problems.

According to the present invention there is provided a treatment apparatus for at least partially disinfecting a fluid such as water, said apparatus comprising: conduit means for conveying a flow of fluid to be treated; at least one ultraviolet (UV) light emitting diode (LED) for the emission of said UV light into said fluid; and control means for controlling said LED; wherein said control means is configured for pulsing said LED at a preselected duty cycle; the or each LED is arranged such that said fluid flows over a surface of each LED, as it is conveyed by the conduit means in operation; and said control means (50) is operable to vary the power and duty cycle of the or each LED continuously; characterised in that: said control means is configured for providing a pulsed control signal to pulse said at least one LED, at a power above the maximum rated power for operating said LED continuously, to create Fourier harmonics during the transition points of the pulsed signal, said harmonics providing different UV frequencies effective for deactivating bacteria, spores, moulds, viruses or the like.

Provision of the conduit means has the advantage that it allows constant circulation of the water being treated, through the treatment apparatus, thereby allowing a greater treatment efficiency than apparatus which relies on the circulation of water around a tank, pond or the like.

The use of an LED makes the use of conduit means practical in small scale environments such as aquariums, ponds or the like. It also allows the provision of a plurality of UV sources in the conduit means, thereby allowing for apparatus having greater treatment efficiency per unit volume of water passed through the conduit means, than with a single UV source. LEDs are also more robust and reliable than UV tubes and lamps, and have the added advantage that the active part of the LEDs are hermetically sealed during manufacture.

Furthermore, since LEDs are relatively low power they are safer to use in an aquatic environment. Thus, the treatment apparatus does not have to meet the same stringent standards as UV lamp based systems.

Arranging the or each LED in the conduit means such that fluid flowing in the conduit flows over a surface of each LED provides for greater treatment efficiency because of the close proximity of each LED to the fluid being treated. It also has the unexpected advantage that the cooling effect of the fluid flow allows each LED to be operated at above its maximum rated power. Operating the or each LED above its maximum rated power allows a higher intensity of UV light to be produced and hence improved treatment capabilities, efficiencies.

Provision of control means configured for pulsing the LED allows the or each LED to be operated at a duty cycle of less than 100% thereby allowing the LED to be operated above its maximum rated power for continuous operation, whilst reducing the overall power consumed, thereby providing enhanced treatment capabilities per unit power consumed. The use of a pulsed signal also improves the treatment efficiency significantly, because the Fourier harmonics produced during the transition points of the pulsed signal provide additional UV frequencies, which contribute significantly to destruction.

Preferably said control means is configured to pulse the or each LED at a duty cycle of less than 100%, More preferably said control means is configured to pulse the or each LED at a duty cycle of less than 50%.

The LED's may be arranged helically about the circumference of the pipe.

The control means is configured to pulse the or each LED at a power above the maximum rated power for operating said LED continuously.

The or each LED may have an emission range having a half intensity emission angle representative of the angular spread of said range. Furthermore the angle between the optical axis of each LED and the longitudinal position of each adjacent axially opposed LED is substantially equal to said emission angle, within a tolerance of 20%. Preferably said angle is between 10° and 45° and more preferably said angle is substantially 30°. The LEDs may be arranged such that the longitudinal distance between each adjacent LED is substantially the minimum distance required to avoid substantial overlap of the emission ranges of axially opposed LEDs.

Preferably said apparatus further comprises a tube within said conduit means, said tube containing a reactive material or a material with a reactive coating to assist purification of the fluid, Alternatively said apparatus may further comprise baffles or plates, included within the conduit means, coated with a metal, a metal oxide or other material that assist purification of the fluid.

Preferably said apparatus further comprises generating means for generating an electromagnetic field in said conduit means (16) to assist purification of said fluid.

Preferably a light emitting portion (22) of each LED extends radially into the conduit (16) through an associated aperture provided in a fluid retaining wall (26) of the conduit, the associated aperture being sealed for fluid impermeability such that the fluid flowing in the conduit, in operation, does not leak out of the conduit through wall (26).

The invention will now be described, by way of example only, with reference to the attached figures in which:
Figure 1 is an illustrative cross-section longitudinally through the centre of a treatment apparatus according to the invention;
Figure 2 is an end view of the treatment apparatus of claim 1, in direction A; and
Figure 3 is a simplified block schematic circuit diagram of circuitry for operation of said apparatus.

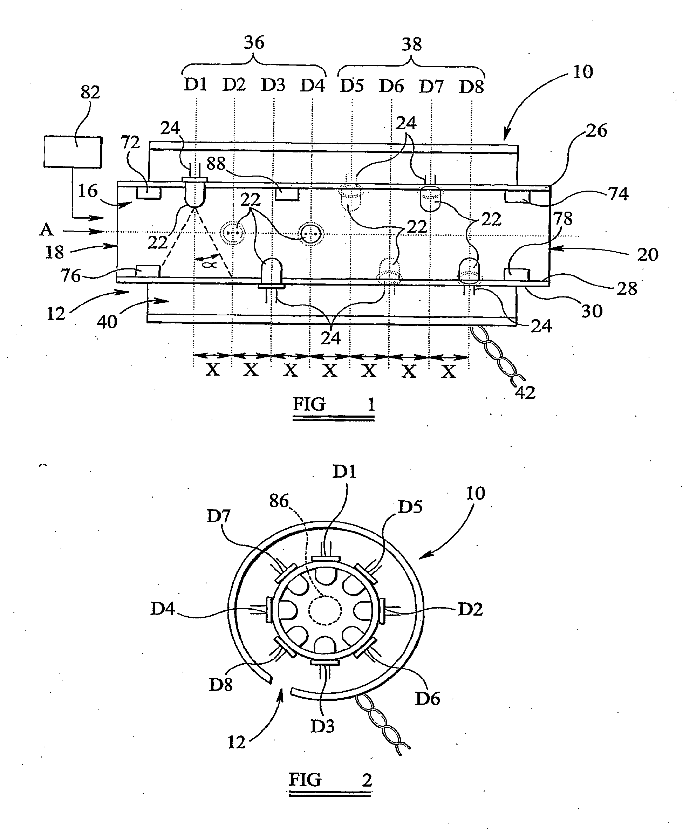

In figures 1 and 2 treatment apparatus for at least partially disinfecting water is shown generally at 10. The apparatus 10 comprises conduit means 12 for conveying a flow of water to be treated, and a plurality of ultra violet (UV) light sources (D1 to D8) for treating the water flowing through the conduit means.

The figures are shown for illustrative purposes only, and are not to scale. The location of all light sources D1 to D8 are shown on figure 1 for the purposes of illustration. In reality D2, D5 and D6 would not be visible in the cross-section.

It will be appreciated that although the apparatus is described with reference to water the apparatus may also be used for treating other fluids, including air, containing organisms or organics. It will be further appreciated that although the apparatus is described with particular reference to applications involving small scale environments such as aquariums or ponds, the apparatus may also be used in other applications, for example, the provision of safe drinking water or the destruction of organisms or organics for environmental applications.

The conduit means 12 comprises a substantially cylindrical pipe 16 or other suitable tube comprising a fluid retaining wall 26 having an internal surface 28 and an external surface 30. The pipe 16 has appropriate dimensions for the treatment application for which the apparatus 10 is intended. For a large aquarium, or small pond, for example, a 20mm diameter pipe is typically appropriate. The pipe 16 may comprise any suitable material but will typically comprise a plastics material, which is resistant to degradation under the effects of UV radiation. The material is also preferably UV reflective, thereby allowing UV light emitted within the pipe 16 to be contained, hence inhibiting potentially hazardous external emission of the UV radiation.

The treatment apparatus 10 is configured for installation as part of a more complex water treatment system. The pipe is configured at an inlet end 18 for sealed onward connection to a source of the water to be treated. In the case of aquatic applications, for example, the apparatus are typically provided with an adaptor (not shown) for onward connection to an aquatic pump (not shown), or the like, which forms part of the more complex system.

The pipe 16 is configured at an outlet end 20 for the onward flow of the water through the system. In aquatic applications such as ponds or aquariums, for example, the outlet end 20 may be configured for direct or indirect onward connection to an aquatic filter or the like. Alternatively or additionally, the outlet end 20 may be configured for the direct or indirect onward flow of water back into an aquarium or pond.

For drinking water purification/sterilisation applications, the pipe 16 may alternatively be configured for interconnection with a water source such as a faucet and/or a drinking water filter.

It will be appreciated that although the pump and filter are described as external components they could alternatively form part of the treatment apparatus 10 itself.

The UV light sources (D1 to D8) each comprises a UV light emitting LED configured to emit light of wavelength between 150nm to 400 nm and preferably 200nm and 400nm, depending on the organisms against which the treatment apparatus is targeted. In general aquatic applications, for example, a wavelength of between 263nm and 275nm is appropriate. Preferably the wavelength is of the order 265nm.

It will be appreciated that although the provision of a plurality of UV light sources is particularly advantageous, a single LED may be used to minimise costs. Where a plurality of LEDs are used, they need not all emit light of the same wavelength. In some applications it may be particularly advantageous to have at least one LED which emits as wavelength of light targeted at deactivation of a particular organism, and at least one other LED which emits a different wavelength of UV light targeted at deactivation of a different organism.

Each LED (D1 to D8) comprises a light emitting portion 22 and an electrical connection portion 24. Each LED (D1 to D8) is arranged such that each light emitting portion 22 extends radially into the pipe 16, through an associated aperture provided in the fluid retaining wall 26. The associated aperture is sealed for fluid impermeability (not illustrated) such that the fluid flowing in the tube, in operation, does not leak out of the pipe 16 through wall 26. The electrical connection portion 24 extends outwardly from the external surface 30 of the wall 26 for onward connection to circuitry for controlling operation of each LED.

Hence, in operation, fluid conveyed by the pipe 16, flows over a surface of the light emitting portion 22 of each LED. The LEDs, therefore, can be self-cleaning LEDs for ease of use.

The LEDs are arranged in first and second groups 36, 38, each comprising four LEDs (D1 to D4 and D5 to D8 respectively). Each LED (D1 to D8) in each group 36, 38 is located at substantially equally spaced longitudinal positions along the pipe 16. Longitudinally adjacent LEDs in each group are located at axially perpendicular positions. The LEDs (D5 to D8) second group 38 is oriented at 45°, relative to the corresponding LEDs (D1 to D4) first group 36, about the longitudinal central axis of the pipe 16. The second group 38 is longitudinally spaced from the first group 36 by a distance substantially equal to the distance between adjacent LEDs in each group 36, 38.

Whilst the arrangement described is particularly advantageous it will be appreciated that alternatively, the LEDs could be arranged helically or even randomly about the circumference of the pipe.

Each LED has a substantially conical half intensity emission range beyond which light emitted from the LED is below the half intensity point relative to light emitted along the optical axis. The half intensity point is characterised by a half intensity angle α indicative of the angular spread of light emitted at the half intensity point.

The longitudinal distance 'x' between each LED (D1 to D8) in each group 36, 38, is selected such that the angle between the optical axis of each LED and the longitudinal position of each LED is substantially equal to the half intensity angle. Typically, for example, the half intensity angle α is 30°, which corresponds to a longitudinal spacing 'x' of approximately 7mm for a 20mm diameter space. Hence, the half intensity range of light emitted by axially opposed LEDs does not substantially overlap, although the half intensity range of adjacent LEDs will partially overlap. Such an arrangement represents optimum longitudinal and radial light coverage within the pipe, and hence improved efficiency.

Electrical circuitry for operation of the LEDs is located on a flexible printed circuit board (PCB) 40 wrapped cylindrically around the pipe 16, as seen in figure 2. Conveniently, the pipe 16 may be provided with a tubular sheath or the like located coaxially around the pipe 16 with the PCB 40 and the electrical connection portions 24 of the LEDs (D1 to D8) being located between the external surface 30 of the pipe 16 and an inner surface of the sheath. The sheath may be implemented using any suitable means but will typically comprise a further tube or pipe of larger diameter than the pipe 16. It will be appreciated that although a flexible circuit board is particularly advantageous in this application, a rigid circuit board could be used.

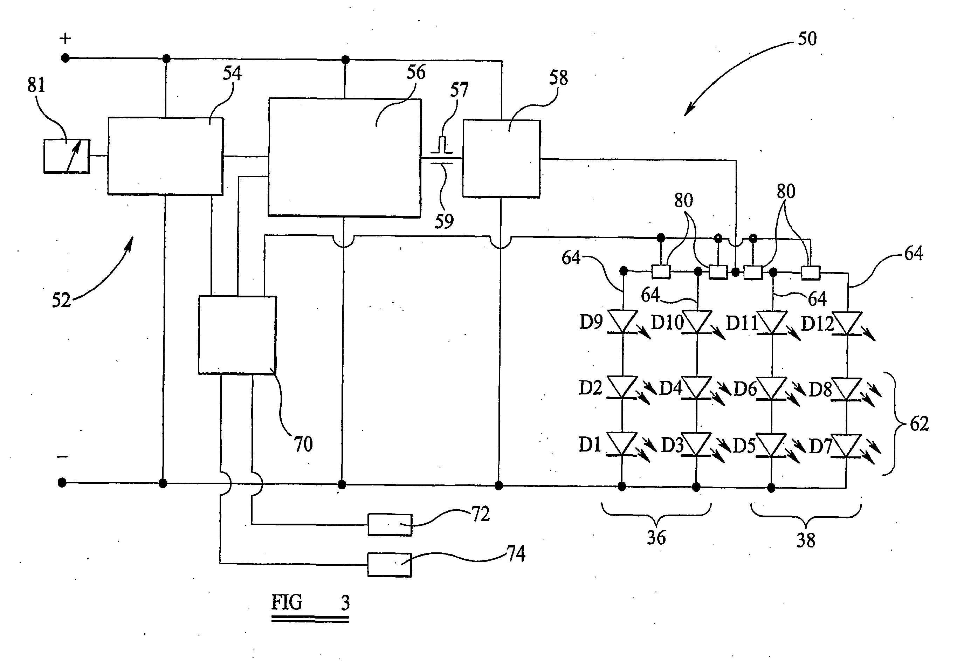

The electronic circuitry on the PCB 40 is electrically interconnected with each connection portion 24 for appropriate operation of the LEDs (D1 to D8) as illustrated in figure 3. The PCB 40 is also provided with means 42 for interconnecting the circuitry to a direct current power supply (not shown).

A simplified block schematic circuit diagram of a circuit including the electronic circuitry on the PCB 40 is shown generally at 50. The circuit 50 comprises an input portion 52, a voltage regulator 54, a manual control 81 for the voltage regulator, a pulse generator portion 56, a control circuit 70, a driver portion 58, an indicator portion 60, sensors 72, 74 and a UV treatment portion 62 comprising the UV treatment LEDs (D1 to D8).

The input portion 52 is configured for interconnection with the DC power supply via connection means 42. The DC power supply may comprise any suitable supply, but typically comprises a 12V battery, or any suitable DC voltage from a mains derived power source.

The voltage regulator 54 is configured to operate off the input DC power, and to provide a regulated output voltage for driving the pulse generator portion 56. The manual control 81 allows the output of the voltage regulator to be manually adjusted if desired.

The pulse generator portion 56 is operable to provide pulsed output signal 57 of variable duty cycle. The pulse generator portion 56 may also be operable to provide a continuous constant voltage output signal 59.

The control circuit 70 controls either or both of the voltage regulator 54 and the pulse generator portion 56 and may be manually adjusted to vary either or both of the output DC voltage from the voltage regulator and the duty cycle of the output from the pulse generator portion 56. These may also be varied in dependence on the signals from the sensors as described further below.

The driver portion 58 is configured to receive, as an input signal, either the continuous signal or the pulsed output signal from the pulse generator portion 56. The driver portion 58 is further configured to produce a substantially constant current pulsed output signal for driving the UV treatment LEDs (D1 to D8) and the indicator portion 60.

The indicator portion 60 comprises a plurality of indicator LEDs (D9 to D12), configured for providing a visual indication, in operation, when the treatment LEDs (D1 to D8) are operating. The indicator LEDs (D9 to D12) are configured for the emission of visible light at wavelengths in excess of the UV range. Typically, for example, the indicator LEDs are configured to emit green or red visible light.

The indicator LEDs and the treatment LEDs are connected in a plurality of parallel circuit branches 64, although it will be appreciated a single circuit branch may be used. Each branch 64 comprises one indicator LED (D9 to D12) and a respective pair of treatment LEDs (D1 to D8). Each indicator LED (D9 to D12), and each diode of the respective pair of treatment LEDs (D1 to D8), in each branch 64, are electrically connected in series, for forward bias, in operation, when the driver portion 58 is providing drive current.

The indicator LEDs (D9 to D12) are arranged for external visibility to a user of the apparatus. The indicator LEDs (D9 to D12) may be arrange on the PCB 40 for external visibility. Alternatively the indicator LEDs (D9 to D12) may be located in any other suitable location, for example, externally on the outer surface of a sheath. Hence, in operation, if one of the UV LEDs fails to operate within the pipe 16, the associated indicator LED will also fail to operate, providing a visual indication that one or more of the LEDs needs to be changed.

The voltage regulator 54, pulse generator 56 and driver portions 58 thus act as control means configured for pulsing the treatment LEDs at a variable duty cycle and at a variable power. Typically, the pulse generator 56 of the control means is configured to produce a pulsed output signal of less than 50% duty cycle. Thus, the treatment LEDs may be operated at a power exceeding their maximum power rating for continuous operation, without significant damage. The cooling effect of fluid flow over the surface of each LED further enhances the maximum current capability, thereby allowing greater treatment efficiency per unit power. The operating power of the treatment LEDs is determined by the constant current output of the driver portion 58. It will be appreciated that when the current output of the driver portion 58 is pulsed and the term 'constant' refers to the current output during each pulse.

At 40% duty cycle, for example, the treatment LEDs may be operated at least one and a half times the maximum power rating. At 10% duty cycle the treatment LEDs may be operated at at least twice the maximum power rating.

The use of a pulsed signal also improves the treatment efficiency significantly, because the Fourier harmonics produced during the transition points of the pulsed signal provide additional UV frequencies, which contribute significantly to destruction of the contaminants. Thus the use of a pulsed signal allows the use of LEDs which emit light having non-optimum wavelengths, without degradation of the treatment capability. For example, an LED which emits UV light having a 400nm wavelength may be used, the harmonics produced enhancing the treatment efficiency beyond that of a continuously operated 270nm LED. At the time of filing, 400nm LEDs are significantly cheaper than 270nm LEDs, hence the cost of the apparatus may be greatly reduced by using pulsed signals.

The beneficial effect of the harmonics increases with reduced duty cycle, thereby further adding to the benefits of using short pulses.

In typical operation the pipe 16 of the conduit means 12 is interconnected for fluid communication between a pump and filter of an existing aquatic system. The treatment LEDs are operated as water is pumped through the pipe 16 over their surfaces. The treatment LEDs are pulsed at an appropriate duty cycle and power taking into account the cooling effect of the fluid flow.

Thus, in operation, the UV light penetrates, for example, green algae cells being carried through the pipe thereby destroying the cells' ability to multiply and causing the cells to flocculate. This flocculation results in larger particles, which can be removed by the filter. Similarly, any organisms carried through the pipe 16 are de-activated. Therefore, the apparatus assists purification by filtration, acts to reduce levels of aquatic bacteria, and reduces levels of Chemical Oxygen Demand (COD) and Total Organic Content (TOC), thereby enhancing water quality.

As is shown in Figure 1, the pipe 16 has sensors 72, 74 positioned at respective ends. Each sensor is provided for monitoring the level of a predetermined contaminant in the water and to provide an output signal in dependence on the level of contaminant. For example, in its simplest form, the sensors 72 can be optical sensors which monitor light emitted from a respective LED 76, 78 and passing through the water. Each sensor 72, 74 is, in this case, conveniently diametrically opposed to the corresponding light source 76, 78. Alternatively, the sensors can monitor the level of UV or natural light passing through the fluid.

The signals from the respective sensors 72, 74 are fed to respective inputs of the control circuit 70 which compares the signals and applies a control signal to the pulse generator portion 56 and/or voltage regulator 54 in dependence on the comparison.

For example, the control circuit 70 can control one or more of the duty cycle, applied voltage and light intensity level of the LEDs in dependence on the comparison. If the level of impurities in the water is relatively light then this will be reflected in the comparison of the signals from the sensors 72, 74 and one or more of the applied voltage from the voltage regulator 54, UV light intensity and the duty cycle can be reduced. Equally, If the level of impurities in the water is relatively high then this will be reflected in the comparison of the signals from the sensors 72, 74 and one or more of the applied voltage from the voltage regulator 54, UV light intensity and the duty cycle can be increased. Thus, the applied voltage, duty cycle and intensity level are chosen individually or in combination to suit the level of impurities or contaminants in the water.

In the absence of any impurities or contaminants the control circuit can regulate the pulse generator and/or voltage regulator such that the LEDs are at an idle setting or off. It is also possible for gates 80 to be provided in the branches to the LEDs such that the control circuit 70 can activate only some of the LED branches for low levels of impurity or contaminant.
It will also be appreciated that the control circuit can vary the duty cycle continuously during operation.

The LEDs can be coated with a material that is transparent to light and also has self-cleaning properties or non-stick properties, eliminating the need to clean the LEDs.

An injection means 82 may be provided for injecting ozone directly into the inlet. This assists in both cleaning the LED surfaces and in purification of the water.

Additional purification means may be included within the pipe 16. One example would be baffles or plates coated with a metal, metal oxide or other material which assists in purification. A further example is a tube 86 which may be of any suitable shape and which contains a reactive material or a material with a reactive coating to assist purification. The tube 86 can be open at the upstream end or at each end to allow flow through of the water or can be perforated or permeable to allow water flow through the tube.

A sound generator 88 can also be provided for transmitting audio or ultrasonic frequencies through the water in the pipe 16 to assist in purification. This can be controlled by the control circuit 70 in a similar manner to the pulse generator portion 56 and gates 80.

In a further modification, an electromagnetic coil can be positioned inside or, preferably coaxially around the outside of the pipe 16 to generate a magnetic field to assist in purification. Again, this can be controlled in the same manner as the pulse generator portion 56.

Finally, the control circuit 70 can be programmed to generate a warning signal to indicate that the complete pipe 16 needs replacing should the signals from the sensors 72, 74 indicate this.

One example of a use of the apparatus according to the invention is in shower heads where water tends to collect in the head. The apparatus can be used with LEDs inside the shower head and the shower head itself forming part or all of the pipe 16.

## Claims

1. Treatment apparatus for at least partially disinfecting a fluid such as water, said apparatus comprising:
conduit means (16) for conveying a flow of fluid to be treated;
at least one ultraviolet (UV) light emitting diode (LED) (D1-D8) for the emission of said UV light into said fluid;
and control means (50) for controlling said LED;
wherein said control means is configured for pulsing said LED at a preselected duty cycle;
and the or each LED is arranged such that said fluid flows over a surface of each LED, as it is conveyed by the conduit means (16) in operation;
said control means (50) is operable to vary the power and duty cycle of the or each LED continuously;
**characterised in that**:
said control means is configured for providing a pulsed control signal to pulse said at least one LED, at a power above the maximum rated power for operating said LED continuously, to create Fourier harmonics during the transition points of the pulsed signal, said harmonics providing different UV frequencies effective for deactivating bacteria, spores, moulds, viruses or the like.

2. Apparatus as claimed in any claim 1 wherein said control means (50) is configured to pulse the or each LED at a duty cycle of less than 100%.

3. Apparatus as claimed in any preceding claim wherein said control means (50) is configured to pulse the or each LED at a duty cycle of less than 50%.

4. Apparatus as claimed in any preceding claim wherein the LED's are arranged helically about the circumference of the pipe.

5. Apparatus as claimed in any preceding claim wherein the or each LED has an emission range having a half intensity emission angle representative of the angular spread of said range.

6. Apparatus as claimed in claim 5 having a plurality of LEDs, wherein the angle between the optical axis of each LED and the longitudinal position of each adjacent axially opposed LED is substantially equal to said emission angle, within a tolerance of 20%.

7. Apparatus as claimed in claim 6 wherein said angle is between 10° and 45°.

8. Apparatus as claimed in claim 6 wherein said angle is substantially 30°.

9. Apparatus as claimed in any of claims 5 to 8 wherein said LEDs are arranged such that the longitudinal distance between each adjacent LED is substantially the minimum distance required to avoid substantial overlap of the emission ranges of axially opposed LEDs.

10. Apparatus as claimed in any of the preceding claims further comprising a tube (86) within said conduit means (16), said tube containing a reactive material or a material with a reactive coating to assist purification of the fluid.

11. Apparatus as claimed in any of the preceding claims further comprising baffles or plates, included within the conduit means (16) coated with a metal, a metal oxide or other material that assist purification of the fluid.

12. Apparatus as claimed in any of the preceding claims further comprising generating means for generating an electromagnetic field in said conduit means (16) to assist purification of said fluid.

13. Apparatus as claimed in any preceding claim wherein a light emitting portion (22) of each LED extends radially into the conduit (16) through an associated aperture provided in a fluid retaining wall (26) of the conduit, the associated aperture being sealed for fluid impermeability such that the fluid flowing in the conduit, in operation, does not leak out of the conduit through wall (26).

## Patentansprüche

1. Behandlungsvorrichtung zum zumindest teilweisen Desinfizieren eines Fluids wie etwa Wasser, wobei die Vorrichtung umfasst:
Leitungsmittel (16) zum Befördern eines Flusses eines zu behandelnden Fluids;
mindestens eine ultraviolettes Licht (UV) emittierende Diode (LED) (D1-D8) zur Emission des UV-Lichts in das Fluid;
und Steuerungsmittel (50) zum Steuern der LED;
worin die Steuerungsmittel zum Pulsen der LED in einem vorgewählten Tastverhältnis konfiguriert sind;
und die oder jede LED so angeordnet ist, dass das Fluid über eine Oberfläche jeder LED fließt, wenn es im Betrieb durch die Leitungsmittel (16) befördert wird;
wobei die Steuerungsmittel (50) betriebsfähig sind, die Leistung und das Tastverhältnis der oder jeder LED kontinuierlich zu variieren;
**dadurch gekennzeichnet, dass**:
die Steuerungsmittel zum Bereitstellen eines gepulsten Steuersignals konfiguriert sind, um die mindestens eine LED mit einer Leistung oberhalb der maximalen Nennleistung für den kontinuierlichen Betrieb der LED zu pulsen, um während der Durchgangspunkte des gepulsten Signals Fourier-Oberschwingungen zu erzeugen, wobei die Oberschwingungen unterschiedliche UV-Frequenzen erzeugen, die zum Deaktivieren von Bakterien, Sporen, Schimmel, Viren oder dergleichen wirksam sind.

2. Vorrichtung nach Anspruch 1, worin die Steuerungsmittel (50) dafür konfiguriert sind, die oder jede LED in einem Tastverhältnis von weniger als 100% zu pulsen.

3. Vorrichtung nach einem der vorhergehenden Ansprüche, worin die Steuerungsmittel (50) dafür konfiguriert sind, die oder jede LED in einem Tastverhältnis von weniger als 50% zu pulsen.

4. Vorrichtung nach einem der vorhergehenden Ansprüche, worin die LEDs schraubenförmig um den Umfang des Rohrs angeordnet sind.

5. Vorrichtung nach einem der vorhergehenden Ansprüche, worin die oder jede LED einen Emissionsbereich hat, der einen Emissionswinkel halber Intensität hat, der die Winkeldivergenz des Bereichs darstellt.

6. Vorrichtung nach Anspruch 5 mit einer Vielzahl von LEDs, worin der Winkel zwischen der optischen Achse jeder LED und der longitudinalen Position jeder benachbarten axial gegenüberliegenden LED im Wesentlichen gleich dem Emissionswinkel ist, innerhalb einer Toleranz von 20%.

7. Vorrichtung nach Anspruch 6, worin der Winkel zwischen 10° und 45° liegt.

8. Vorrichtung nach Anspruch 6, worin der Winkel im Wesentlichen 30° beträgt.

9. Vorrichtung nach einem der Ansprüche 5 bis 8, worin die LEDs so angeordnet sind, dass der longitudinale Abstand zwischen jeder benachbarten LED im Wesentlichen der Mindestabstand ist, der erforderlich ist, um eine wesentliche Überschneidung der Emissionsbereiche axial gegenüberliegender LED zu vermeiden.

10. Vorrichtung nach einem der vorhergehenden Ansprüche, ferner einer Röhre (86) innerhalb der Leitungsmittel (16) umfassend, wobei die Röhre ein reaktionsfähiges Material oder ein Material mit einer reaktionsfähigen Beschichtung enthält, um die Reinigung des Fluids zu unterstützen.

11. Vorrichtung nach einem der vorhergehenden Ansprüche, ferner in die Leitungsmittel (16) einbezogene Leitbleche oder Platten umfassend, die mit einem Metall, einem Metalloxid oder einem anderen Material beschichtet sind, welche die Reinigung des Fluids unterstützen.

12. Vorrichtung nach einem der vorhergehenden Ansprüche, ferner Erzeugungsmittel zum Erzeugen eines elektromagnetischen Feldes in den Leitungsmitteln (16) umfassend, um die Reinigung des Fluids zu unterstützen.

13. Vorrichtung nach einem der vorhergehenden Ansprüche, worin ein Licht emittierender Abschnitt (22) jeder LED sich durch eine zugeordnete Öffnung, die in einer Fluidhaltewand (26) der Leitung vorgesehen ist, radial in die Leitung (16) hinein erstreckt, wobei die zugeordnete Öffnung für Fluidundurchlässigkeit abgedichtet ist, sodass das in der Leitung fließende Fluid im Betrieb nicht durch die Wand (26) aus der Leitung austritt.

## Revendications

1. Appareil de traitement pour désinfecter au moins partiellement un fluide tel que de l'eau, ledit appareil comprenant :
un moyen de canalisation (16) permettant d'acheminer un flux de fluide à traiter ;
au moins une diode électroluminescente (DEL) (D1 à D8) émettant un ultraviolet (UV) en vue d'une émission de ladite lumière ultraviolette dans ledit fluide ;
et un moyen de commande (50) permettant de commander ladite DEL ;
dans lequel ledit moyen de commande est configuré pour faire pulser ladite DEL selon un cycle de service présélectionné ;
et la ou chaque DEL est agencée de telle manière que ledit fluide s'écoule sur une surface de chaque DEL, à mesure qu'il est acheminé par le moyen de canalisation (16) en cours de fonctionnement ;
ledit moyen de commande (50) peut servir à faire varier la puissance et le cycle de service de la ou de chaque DEL de manière continue ;
**caractérisé en ce que** :
ledit moyen de commande est configuré pour fournir un signal de commande pulsé afin de faire pulser ladite au moins une DEL à une puissance supérieure à la puissance maximale calibrée pour faire fonctionner ladite DEL de manière continue, afin de créer des harmoniques de Fourier pendant les points de transition du signal pulsé, lesdites harmoniques fournissant différentes fréquences UV efficaces pour détruire des bactéries, des spores, des moisissures, des virus ou similaires.

2. Appareil selon la revendication 1, dans lequel ledit moyen de commande (50) est configuré pour faire pulser la ou chaque DEL selon un cycle de service inférieur à 100 %.

3. Appareil selon l'une quelconque des revendications précédentes, dans lequel ledit moyen de commande (50) est configuré pour faire pulser la ou chaque DEL selon un cycle de service inférieur à 50 %.

4. Appareil selon l'une quelconque des revendications précédentes, dans lequel les DEL sont agencées de manière hélicoïdale autour de la circonférence d'une conduite.

5. Appareil selon l'une quelconque des revendications précédentes, dans lequel la ou chaque DEL présente une plage d'émission présentant un angle d'émission de demi-intensité représentatif de la dispersion angulaire de ladite plage.

6. Appareil selon la revendication 5, présentant une pluralité de DEL, dans lequel l'angle compris entre l'axe optique de chaque DEL et la position longitudinale de chaque DEL axialement opposée adjacente est essentiellement égal audit angle d'émission, à l'intérieur d'une tolérance de 20 %.

7. Appareil selon la revendication 6, dans lequel ledit angle est compris entre 10° et 45°.

8. Appareil selon la revendication 6, dans lequel ledit angle est essentiellement de 30°.

9. Appareil selon l'une quelconque des revendications 5 à 8, dans lequel lesdites DEL sont agencées de telle manière que la distance longitudinale entre chaque DEL adjacente est essentiellement la distance minimum requise pour éviter un chevauchement substantiel des plages d'émission de DEL axialement opposées.

10. Appareil selon l'une quelconque des revendications précédentes, comprenant en outre un tube (86) au sein dudit moyen de canalisation (16), ledit tube contenant un matériau réactif ou un matériau avec un revêtement réactif afin de contribuer à une purification du fluide.

11. Appareil selon l'une quelconque des revendications précédentes, comprenant en outre des chicanes ou des plaques, incluses dans le moyen de canalisation (16) et revêtues d'un métal, d'un oxyde métallique ou d'un autre matériau qui contribuent à une purification du fluide.

12. Appareil selon l'une quelconque des revendications précédentes, comprenant en outre un moyen de génération permettant de générer un champ électromagnétique dans ledit moyen de canalisation (16) afin de contribuer à une purification dudit fluide.

13. Appareil selon l'une quelconque des revendications précédentes, dans lequel une partie électroluminescente (22) de chaque DEL s'étend radialement dans la canalisation (16) à travers une ouverture associée fournie dans une paroi de rétention de fluide (26) de la canalisation, l'ouverture associée étant fermée de manière étanche en vue d'une imperméabilité au fluide de telle manière que, en cours de fonctionnement, le fluide s'écoulant dans la canalisation ne fuit pas hors de la canalisation à travers la paroi (26).
